# EUROPEAN PATENT SPECIFICATION

(11) **EP 1 224 159 B1**
(45) Date of publication and mention of the grant of the patent: **25.02.2004**
(21) Application number: 00969235.1
(22) Date of filing: 20.10.2000
(51) Int. Cl.: C07C 209/62, C07C 209/26, C07C 209/70, C07C 211/42, C07C 251/20

(54) **IMPROVED SYNTHESIS OF RACEMIC SERTRALINE**
RACEMISCH SERTRALIN SYNTHESE
SYNTHESE AMELIOREE DE SERTRALINE RACEMIQUE

(30) Priority: 27.10.1999 DK 154099
(43) Date of publication of application: 24.07.2002
(73) Proprietor: A/S GEA Farmaceutisk Fabrik, 2650 Hvidovre (DK)
(72) Inventor: FISCHER, Erik, DK-2300 Copenhagen S (DK); TREPPENDAHL, Svend, Peter, DK-2830 Virum (DK); PEDERSEN, S ren, Bols, DK-2650 Hvidovre (DK)
(74) Representative: Simonsen, Christian Rosendal
(86) International application number: PCT/DK2000/000586
(87) International publication number: WO 2001/030742

(56) References cited:
- EP-A- 0 947 499
- WO-A-93/01161
- US-A- 4 536 518

## Description

The present invention relates to a new improved process for producing cis-(1S),(4S)-N-methyl-4-(3,4-dichlorophenyl)-1,2,3,4-tetrahydro-1-naphthaleneamine, hereinafter referred to as "sertraline", or salts thereof, and to new intermediates in the process.

### BACKGROUND OF THE INVENTION

Sertraline hydrochloride is a valuable pharmaceutically active substance which is widely used for treatment of depressions and anxiety disorders.

In DK 153 390 a process for the production of sertraline is described where a key intermediate 4-(3,4-dichlorophenyl)-3,4-dihydro-1(2H)-naphthalenone, hereinafter referred to as "tetralone", is condensed with an amine having the formula HNR₁R₂, wherein R₁ is hydrogen or n-alkyl with 1-3 carbon atoms, and R₂ is n-alkyl with 1-3 carbon atoms, in the presence of titanium tetrachloride to the corresponding N-methyl-imine. Subsequently the N-methyl-imine is hydrogenated, using palladium on carbon as catalyst, to a mixture of cis and trans isomers of N-methyl-4-(3,4-dichlorophenyl)-1,2,3,4-tetrahydro-1-naphthaleneamine. The racemic cis-isomer is separated from the trans-isomer by crystallisation of the cis-isomer as a hydrochloride from a methanol or ethanol solution. Finally the racemic cis-isomer is resolved by a diastereomeric crystallisation with D-(-)-mandelic acid in order to obtain the desired stereoisomer cis- (1S) , (4S) -N-methyl-4- (3, 4-dichlorophenyl)-1,2,3,4-tetrahydro-1-naphthaleneamine, sertraline.

The cis/trans ratio obtained in the hydrogenation step is 70/30 which results in an overall yield of 68 % racemic cis-N-methyl-4-(3,4-dichlorophenyl)-1,2,3,4-tetrahydro-1-naphthaleneamine.

WO 93/01161 describes a process for the production of trans-N-alkanoyl-N-methyl-4-(3,4-dichlorophenyl)-1,2,3,4-tetrahydro-1-naphthaleneamine, from the corresponding 4-hydroxy precursor. EP 594 666 describes the formation of the cis-N-alkanoyl-N-methyl-4-(3,4-dichlorophenyl)-1,2,3,4-tetrahydro-1-naphthaleneamine by elimination and subsequent catalytic hydrogenation. Further EP 594 666 describes that the alkanoyl group can be removed to form cis isomer i.e. racemic sertraline.

In WO 98/27050 a process for the preparation of racemic cis-N-methyl-4-(3,4-dichlorophenyl)-1,2,3,4-tetrahydro-1-naphthaleneamine is described, wherein tetralone is reacted with N-methyl-hydroxylamine to produce N-methyl-4-(3,4-dichlorophenyl)-1,2,3,4-tetrahydro-1-naphthalene-1-en-amine-N-oxide which is hydrogenated in the presence of a catalyst to form N-methyl-4-(3,4-dichlorophenyl)-1,2,3,4-tetrahydro-1-naphthaleneamine. The cis isomer is separated by precipitation. A cis/trans ratio of 80/20 and an overall yield of 69 % racemic cis-isomer is obtained.

The above processes provide sertraline starting from tetralone. Tetralone may be produced in a 5-step process as described in DK 153 390, with benzoyl chloride as starting material.

DK 171 020 describes a procedure where an α-naphthol is reacted with ortho-dichlorobenzene to produce a 4(di-substituted phenyl)-1-tetralone.

WO 95/15299 describes a process for the preparation of (4S)-4-(3,4-dichlorophenyl)-3,4-dihydro-1(2H)-naphthalenone where racemic 4-(3,4-dichlorophenyl)-3,4-dihydro-1(2H)-naphthalenone is reduced using an asymmetric reagent to produce a mixture of cis and trans alcohols, said cis alcohols are separated from the trans alcohols and oxidized to the desired (4S) enantiomers.

In WO 98/15516 a process for producing 4-(3,4-dichlorophenyl)-3,4-dihydro-1(2H)-naphthalenone by reaction of o-dichloro-benzene and α-naphthol in the presence of a Fridel-Craft catalyst is described. From the reaction mixture the 4-(3,4-dichlorophenyl)-3,4-dihydro-1(2H)-naphthalenone is separated from another reaction product 4-(2,3-dichlorophenyl)-3,4-dihydro-1(2H)-naphthalenone by a number of crystallisation steps.

A process for production of the (4S)-enantiomer of 4-(3,4-dichlorophenyl)-3,4-dihydro-1(2H)-naphthalenone in a highly optical-pure form is described in WO 93/12062. In this process a 4-(3,4-dichlorophenyl)-4-ketobutanoic acid is first esterified with isopropylene or isobutylene. The ester is in three steps converted to isopropyl- or tert.- butyl- 4-(3,4-dichlorophenyl)-(4R)-phenylbutanoate which is cyclizised in a final step to form the desired (4S)-4-(3,4-dichlorophenyl)-3,4-dihydro-1(2H)-naphthalenone.

In spite of the several prior art processes discussed above, there is still a desire of improving the process for production of sertraline in order to make the process more cost efficient and more environmentally acceptable. In the process used industrially the overall yield of racemic sertraline from tetralone is only approximately 68-69% which means that approximately 30 % is lost in the process and in one way or another forms a waste product that needs to be handled. In addition there is a wish to avoid the usage of titanium tetrachloride because of the difficulties of handling this compound and the titanium containing waste that is formed during the process.

Thus it is an object of the present invention to provide a new process for producing racemic sertraline from tetralone in a simple, economical and environmentally acceptable way.

### SUMMARY OF THE INVENTION

The present invention is based on the recognition that the yield can be improved by ensuring that a higher cis/trans ratio is obtained, and that an advantageously high cis/trans ratio is favoured when the reduction of a compound formed by reaction of tetralone and an amine contains a "bulky" substituent on the nitrogen atom instead of a small substituent such as a methyl group as used in the prior art. The required methyl substituent on the N-atom in the final product, sertraline, is added after the formation of the desired conformation at the 1-carbon. Eventually the "bulky" group is removed.

Thus, in one aspect it is an object of the present invention to provide a process for the production of cis-(1S), (4S)-N-methyl-4-(3,4-dichlorophenyl)-1,2,3,4-tetrahydro-1-napthaleneamine, sertraline, or a salt thereof, which process comprises the following steps:
i) the compound with the formula I 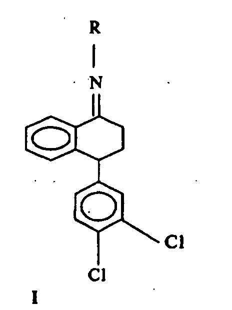 Wherein R = a benzyl group, which may be substituted with one to three groups independently selected from: fluorine, chlorine, bromine, iodine, alkyl groups such as methyl, ethyl and propyl, and alkoxy groups such as methoxy, ethoxy, and propoxy,
   is hydrogenated to obtain the compound with the formula II, 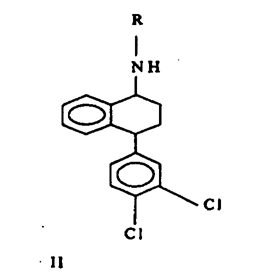 wherein R is as defined above,
ii) the compound with the formula II is reductively alkylated with formaldehyde or a derivate of formaldehyde or reacted with another methylating agent to obtainthe compound with the formula III, 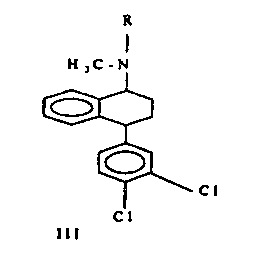 wherein R is as defined above, and
iii) the compound with the formula III is converted to sertraline or a salt thereof, by removal of group R.

Detailed explanation of the process of the invention for removing the group R from the compound (III) may be found below.

The compound (I) may be obtained by reaction of 4-(3,4-dichlorphenyl)-3,4-dihydro-1(2H)-naphthalenone tetralone, with R-NH₂ where R has the above meaning. This reaction may be performed without use of catalyst especially without use of the environmentally hazardous titanium tetrachloride.

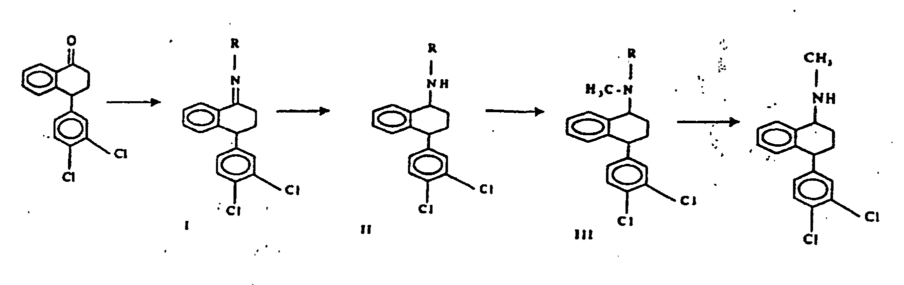

The compound of formula I, wherein R has the above significance is novel and in a further aspect of the invention forms part thereof.

The intermediate compound (II) may be obtained by hydrogenation of a compound of the formula I.

Neither the compound (II) has been described previously and thus the invention in a still further aspect comprises the novel compound of formula II wherein R is as defined above.

The hydrogenation of compound (I) to compound (II) is preferably performed in the presence of a solid catalyst. In this step the configuration at the 1-carbon atom is determined.

The substituent on the nitrogen atom influences the cis/trans ratio in the compound II, and a substituent as R favours a high cis/trans ratio compared to the ratio when a smaller substituent such as a methyl group is present in stead of R. The high cis/trans ratio is maintained in the subsequent two steps for forming sertraline.

The compound (III) is manufactured by methylation of a compound of the formula (II), and eventually converted into sertraline by removal of the group R.

The methyl group introduced by the conversion of compound II into the compound III remains attached to the nitrogen atom when the latter compound is converted into the desired compound, sertraline.

Neither the intermediate compound (III) wherein R has the above significance, has been previously described and forms a further aspect of the invention.

Thus the present invention provides a process and intermediates for production of racemic cis-N-methyl-4-(3,4-dichlorophenyl)-1,2,3,4-tetrahydro-1-naphthaleneamine in a surprisingly high yield when calculated on the starting material compound (I) or even "tetralone". This is because the cis/trans ratio for the reaction is very high and also because the conversion rate in the total process is high.

In addition the procedure is simple, inter alia because the conversion of (I) to (II) and (II) to (III) may be performed in one pot without the need for intermediate purification operations.

The process is gentle to the environment as it can be performed without the need for titanium tetrachloride. In addition it can be run cost efficiently as the yield is higher than the yield in previous known processes, the use of expensive compounds can be avoided, and excess R-NH₂ for the formation of (I), may be removed from the reaction mixture by distillation and reused.

### DETAILED DESCRIPTION OF THE INVENTION

### Conversion of "tetralone" to compound I

For the synthesis of sertraline the compound I may be formed by reacting 4-(3,4-dichlorophenyl)-3,4-dihydro-1(2H)-naphthalenone with an amine of the formula R-NH₂.

The reaction may be performed in any suitable solvent that is inert with respect to the reaction or it may be performed without a solvent. As water is produced during the reaction it is preferred to use a solvent that can remove the water by azeotropical distillation. Examples of such solvents are cyclohexane, benzene and toluene.

The temperature for the reaction can be selected between room temperature and the boiling temperature of the solvent. It is preferred to perform the reaction at a temperature in the interval 50-200°C, preferably between the boiling point of the azeotrope of water and solvent and the boiling temperature of the solvent.

The reaction can be performed without any catalyst or can be performed with an acid catalyst such as a carboxylic acid e.g. capronic acid. After the reaction is finished the solvent and excess amine may be removed by distillation and reused.

In connection with the group R the term "bulky" means that the group is large. Consequently the group R may be selected among large groups that is substantially inert to the reaction in question. In this context large means that the group has a size substantially larger that the methyl group attached to the N-atom in prior art procedures. Usable groups R according to this invention may be selected within the above definition.

As examples of groups R for use according to this invention can be mentioned: benzyl, o-chlorobenzyl, m-chlorobenzyl, p-chlorobenzyl, p-flourobenzyl, 2,3-dichlorobenzyl, 2,4-dichlorobenzyl, 2-bromo-4-chlorobenzyl, p-methoxybenzyl, 2,3-dimethoxybenzyl, 2,4-dimethoxybenzyl, 3,4-dimethoxybenzyl, 2,3,5-trimethoxybenzyl, 4-ethylbenzyl.

In principle there are rather few limitations on R, since the group is removed before the final product is achieved. However it is preferred to select R so that R-NH₂ has a convenient boiling point, i.e. it is not distilled off during reaction but can easily removed by distillation after the reaction.

For industrial purposes it is preferred to use a compound that is available at a low price. For this reason it is preferred to use benzylamine which is both quite reactive and very cheap, and gives N-[4-(3,4-dichlorophenyl)-3,4-dihydro-1(2H)-naphthalenylidene]-benzylamine as high melting crystals in a surprisingly high yield.

### Conversion of compound I to compound II

The reduction of compound I to compound II is believed to be the step that determines the cis/trans ratio for the final product.

The reduction may take place in several ways as it will be known to the person skilled in the art. The reduction may be performed using complex hydrides (e.g. NaBH₄) or by hydrogenation. Reduction performed by catalytic hydrogenation tends to give better selectivity that reduction using the complex hydrides. For example aliquots of N-[4-(3,4-dichlorophenyl)-3,4-dihydro-1(2H)-naphthalenylidene]-benzylamine were reduced with NaBH₄ and Raney nickel/H₂ respectively, and subsequently reductively alkylated with formaldehyde, where after the cis/trans ratio was analyzed. The result was a ratio of 53,8/46,2 using NaBH₄ compared to 82,9/17,1 for Raney Nickel/H₂ which clearly demonstrates the selectivity of the catalytic hydrogenation. An even higher selectivity with a cis/trans ratio of 93,5/6,5 has been observed using palladium on carbon.

For the purpose of this invention the catalytic hydrogenations can be performed using a solid catalyst as known within the art, such as palladium on carbon, rhodium on carbon and Raney nickel. The reaction can be performed in any solvent that is inert to the reaction e.g. ethanol. As it will be known within the art hydrogenations using H₂ in the presence of a solid catalyst gives varying results depending on the actual used catalyst, particle size, pressure, temperature etc. It is within the capabilities of the skilled person in the art to determine which conditions gives the optimal result in each actual reaction.

### Conversion of compound II to III

The conversion of II to III is conveniently carried out in the same pot as the reaction of I to II.

The reaction takes place simply by addition of a solution of formaldehyde to the reaction mixture from the previous step. The reaction takes place almost instantaneously and is almost quantitative.

In stead of formaldehyde, any derivative of formaldehyde such as paraformaldehyde, and acetals and hemiacetals thereof may be used.

The compound III may also be made by alkylation of compound II by conventional alkylation using for example methyl iodide. The reaction is fast and high yielding but requires the isolation of the starting material which may be considered inconvenient.

As in the previous step any solvent that is inert to the reaction may be used.

### Conversion of compound III to racemic Sertraline

When the compound III has been made it is converted to racemic cis-N-methyl-4-(3,4-dichlorophenyl)-1,2,3,4-tetrahydro-1-naphthaleneamine by removal of the group R according to the invention using any procedure known within the art, see for example Theodora W.Greene and Peter G.M. Wuts: Protective Groups in Organic Synthesis. 2. ed, p.364-366, John Wiley & sons, inc. 1991).

Thus for the use within this invention removal can e.g. be performed by hydrogenation in acidic environment or by treatment with an acid. Hydrogenation is preferred because R groups suitable for acid cleavage like for instance triphenylmethyl, 5-benzosuberyl, di(4-methoxyphenyl)methyl, 3,4-dimethylbenzyl are all very expensive to introduce compared to the simple benzyl group. Hydrogenation may take place in a inert solvent such as ethanol, in the presence of a solid catalyst and an acid, such as acetic acid or hydrochloric acid. Thus performing the debenzylation of N-benzyl-N-methyl-4-(3,4-dichlorophenyl)-1,2,3,4-dihydro-1-naphthaleneamine in ethanol with acetic acid leads to about 80% of the cis and trans sertraline racemates and about 20 % of the 4-(3,4-dichlorophenyl)-1,2,3,4-tetrahydro-naphthalene.

The use of hydrochloric acid results in precipitation of the formed racemic cis-N-methyl-4-(3,4-dichlorophenyl)-1,2,3,4-tetrahydro-1-naphthaleneamine as a hydrochloride which leads to an almost complete conversion of the compound III to racemic sertraline hydrochloride. Surprisingly, the precipitation of the cis-N-methyl-4-(3,4-dichlorophenyl)-1,2,3,4-tetrahydro-1-naphthaleneamine hydrochloride does not influence the hydrogenation in a negative way indicating that no clotting of the catalyst occurs.

### Conversion of racemic sertraline to sertraline

The formed racemic cis-N-methyl-4-(3,4-dichlorophenyl)-1,2,3,4-tetrahydro-1-naphthaleneamine is separated from the reaction mixture using known procedures, such as crystallisation of cis-N-methyl-4-(3,4-dichlorophenyl)-1,2,3,4-tetrahydro-1-naphthaleneamine as a hydrochloride by addition of hydrochloric acid followed by filtration of the crystals from the mixture.

In case that hydrochloride was used in the previous step and racemic cis-N-methyl-4-(3,4-dichlorophenyl)-1,2,3,4 -tetrahydro-1-naphthaleneamine is present in form of a precipitate, the precipitate and the solid catalyst are removed from the solution e.g. by filtration.

The purified racemic cis-N-methyl-4-(3,4-dichlorophenyl)-1,2,3,4-tetrahydro-1-naphthaleneamine hydrochloride may be resolved by known procedures such as a diastereomeric crystallisation with D-(-)-mandelic acid, and subsequent conversion to the hydrochloride to produce the pharmaceutical desired form cis-(1S),(4S)-N-methyl-4-(3,4-dichlorophenyl)-1,2,3,4-tetrahydro-1-naphthaleneamine hydrochloride, sertraline hydrochloride.

### EXAMPLES

### Example 1.

### N-[4-(3,4-dichlorophenyl)-3,4-dihydro-1(2H)-naphthalenylidine]benzylamine, (Formula I, R=Benzyl).

4-(3,4-dichlorophenyl)-3,4-dihydro-1(2H)-naphthalenone ("tetralone") (100.0 g, 0.343 mole) and benzylamine (70.0 g, 0.653 mole) was refluxed in 200 ml toluene using a Dean-Stark water separator. When the theoretical amount of water (6.2) ml was trapped (7 hours), toluene and the excess benzylamine was distilled off *in vacuo* at 90°C. 250 ml 2-propanol was added and the suspension was heated to reflux. The solution was cooled to 5°C and stirred for 12 hours whereby crystals were formed. N-[4-(3,4-dichlorophenyl)-3,4-dihydro-1(2H)-naphthalenylidene]benzylamine was removed by filtration and washed with 100 ml of cold 2-propanol and dried *in vacuo,* leaving 124.7 g (96 %) of N-[(3,4-dichlorophenyl)-3,4-dihydro-1(2H)-naphthalenylidine]-benzylamine as light brown crystals.
Melting point: 102-103°C
¹H-NMR (CDCl₃, 300 MHz) δ ppm: 8.40 (m, 1H); 7.30 (m, 9H); 6.88 (m, 2H); 4.68 (s, 2H); 4.15 (m, 1H); 2.60 (t, 2H) ; 2.25 (m, 1H) ; 2.15 (m, 1H).

### Example 2.

### N-methyl-N-benzyl-4-(3,4-dichloro-phenyl)-1,2,3,4-tetrahydro-1-naphthaleneamine, (Formula III, R=benzyl).

A one litre autoclave was charged with N-[4-(3,4-dichlorophenyl)-3,4-dihydro-1(2H)-naphthalenylidine]-benzylamine (50.0 g, 0,132 mole), 10 ml triethylamine, 500 ml ethanol and 7.0 g Raney-nickel (BK 111 W, Degussa) washed dry with ethanol. The suspension was hydrogenated at a pressure of 3 bar at 60 °C until hydrogen uptake ceased (3 hours). A sample of the solution was analyzed using HPLC on a nucleosil C18 column, eluted using MeOH/phosphate buffer, pH 7.0 (9/1) with a flow of 0.9 ml/min and UV detection at 230 nm. A single product with a Rt=11.8 min was observed.

The synthesis was continued by addition of 25 ml of 37 % formaldehyde to the solution and hydrogenation at a pressure of 3 bar at 60°C was resumed. When hydrogen uptake ceased the hot solution was filtered and evaporated *in vacuo* to approximately 150 ml. 150 ml of toluene and 150 ml of water was added and the organic layer was isolated. The aqueous phase was extracted with 100 ml of toluene and the organic layers were combined and washed with 50 ml of water and evaporated *in vacuo* leaving 54.1 g of a light brown oil. HPLC analysis ( nucleosil C18 column eluted using MeCN/phosphate buffer, pH 3.0 (1/1) with a flow of 1.9 ml/min and UV detection at 230 nm ) revealed two products with Rt=14.53 min ( cis racemate: 82.9%) and Rt=12.85 min (trans racemate: 17.1 %).

### Example 3.

### N-methyl-4-(3,4-dichlorophenyl)-1,2,3,4-tetrahydro-1-naphthaleneamine, (cis-racemate, racemic sertraline).

A one litre autoclave was charged with 54.1 g (0,132 mole ) of the light brown oil from example 2, 400 ml of ethanol, 14 ml (0,168 mole) of concentrated hydrochloric acid and 2.5 g of palladium on carbon 10 %. The suspension was hydrogenated at a pressure of 3 bar at room temperature until hydrogen uptake ceased (5 hours). The resulting grey suspension of precipitated cis-racemate and catalyst was made alkaline with concentrated NaOH (20.0 ml; 0.22 mole), toluene was added and the mixture was stirred for 30 minutes. HPLC analysis (nucleosil C18 column eluted using MeOH/phosphate buffer, pH 7.0 (9/1) with a flow of 0.7 ml/min and UV detection at 230 nm) revealed two products with RT= 14.01 min (cis-racemate) and RT= 16.36 min (trans-racemate) where the cis/trans ratio was 80.4/19.8. The catalyst was removed by filtration and toluene (200 ml) and water (600 ml) was added to the filtrate. The organic layer was separated and the aqueous layer washed with toluene (100 ml).

The combined organic extracts were washed with water (40 ml) and brine (20 ml) and the solvent was evaporated *in vacuo* leaving 47.5 g of a light brown oil. The oil was taken up in ethanol (250 ml), 25 ml of 6.28 M hydrochloric acid (0.157 mole) in ethanol was added and the solution was stirred for 3 hours at 0°C. The cis racemate immediately crystallized. The white solid was separated by filtration, washed with 80 ml of ethanol at -10°C and dried *in vacuo* at 50°C over night leaving 36.1 g of cis-N-methyl-4-(3,4-dichlorophenyl)-1,2,3,4-tetrahydro-1-naphthaleneamine as a white solid having a melting point of 288-290°C (uncorr.). The yield was calculated to 80.2% based on N-[4-(3,4-dichlorophenyl)-3,4-dihydro-1(2H)-naphthalenylidine]-benzylamine. The overall yield based on tetralone was 77.0 %.

### Example 4

### N-[4-(3,4-dichlorophenyl)-3,4-dihydro-1(2H)-naphthalenylidine]-4-chlorobenzylamine, (Formula I, R=p-chlorobenzyl)

4-(3,4-dichlorophenyl)-3,4-dihydro-1(2H)-naphthalenone ("tetralone") was reacted with p-chlorobenzyl amine according to the procedure outlined in example 1, and N-[4-(3,4-dichlorophenyl)-3,4-dihydro-1(2H)-naphthalenylidene]-4-chlorobenzylamine was obtained as brown crystals melting at 116-117°C, in a yield of 89.4 %.

### Example 5

### N-[4-(3,4-dichlorophenyl)-3,4-dihydronaphthalenylidine]-4-methoxybenzylamine, (Formula I, R=p-methoxybenzyl)

4-(3,4-dichlorophenyl)-3,4-dihydro-1(2H)-naphthalenone ("tetralone") was reacted with p-methoxybenzyl amine according to the procedure outlined in example 1, and N-[4-(3,4-dichlorophenyl)-3,4-dihydro-1(2H)-naphthalenylidene]-4-methoxybenzylamine was obtained as brown crystals melting at 107-108°C, in an yield of 85.6 %.

### Example 6

### N-[4-(3,4-dichlorophenyl)-3,4-dihydro-1(2H)-naphthalenylidene]-3,4-dimethoxy-benzylamine, (Formula I, R=3,4-dimethoxy-benzyl).

4-(3,4-dichlorophenyl)-3,4-dihydro-1(2H)-naphthalenone ("tetralone") was reacted with 3,4-dimethoxy-benzyl amine according to the procedure outlined in example 1, and N-[4-(3,4-dichlorophenyl)-3,4-dihydro-1(2H)-naphthalenylidene]-3,4-dimethoxybenzylamine was obtained as colourless crystals melting at 121-122°C in an yield of 92.3 %.

## Claims

1. Process for the produdction of cis-(1S), (4S)-N-methyl-4-(3,4-dichlorphenyl)-1,2,3,4-tetrahydro-1-naphtaleneamine, sertraline, or a salt thereof **characterized in that** the process comprises the following steps:
i) the compound with the formula I 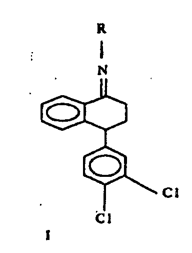 wherein R = a benzyl group, which may be substituted with one to three groups independently selected from: fluorine, chlorine, bromine, iodine, alkyl groups such as methyl, ethyl and propyl, and alkoxy groups such as methoxy, ethoxy, and propoxy,
is hydrogenated to obtain the compound with the formula II 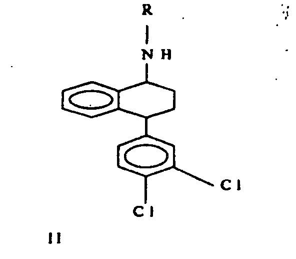
ii) the compound with the formula II is reductively alkylated with formaldehyde or a derivative of formaldehyde to obtain the compound with the formula III, or the compound with the formula II is alkylated with a methylating agent to obtain the compound with the formula III 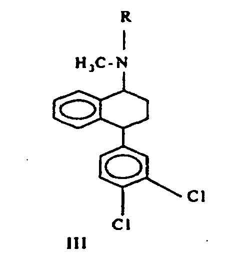
iii) the compound with the formula III is converted to sertraline or a salt thereof by removal of the group R.

2. Process according to claim 1, wherein the formaldehyde or a derivate of formaldehyde is selected from the group consisting of formaldehyde, paraformaldehyde, acetals and hemiacetals thereof.

3. Process according to claim 1, wherein the methylating agent is methyl iodide.

4. Process according to any of claims 1-3, wherein step i) is performed in the presence of a catalyst selected from palladium on carbon, rhodium on carbon and Raney nickel.

5. Process according to any of claims 1-4, wherein step i) and step ii) are performed in the same pot without intermediate purification.

6. Process according to any of claims 1-5, wherein step iii) is performed by hydrogenolysis.

7. Process according to any of claims 1-6, wherein R = benzyl.

8. An N-substituted N-[4-(3,4-dichlorophenyl)-3,4-dihydro-1(2H)-naphthalenylidine)-amine of the formula I, wherein R is defined in claim 1.

9. An N-substituted N-(3,4-dichloro-phenyl)-1,2,3,4-tetrahydro-1-naphthaleneamine of the formula II, wherein R is defined in claim 1.

10. An N-substituted N-methyl-4-(3,4-dichlorophenyl)-1,2,3,4-tetrahydro-1-naphthaleneamine of the formula III, wherein R is defined in claim 1.

## Patentansprüche

1. Verfahren zur Herstellung von Cis-(1S), (4S)-N-methyl-4-(3,4-dichlorphenyl)-1,2,3,4-tetrahydro-1-naphtalenamin, Sertralin oder eines Salzes davon, **dadurch gekennzeichnet, dass** das Verfahren folgende Stufen umfasst:
i) die Verbindung der Formel I 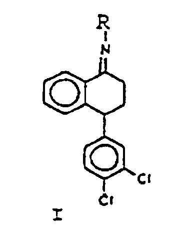 in welcher R eine Benzylgruppe ist, die mit 1-3 unabhängig aus Fluor, Chlor, Brom, Jod, Alkylgruppen wie etwa Methyl, Ethyl und Propyl, und Alkoxygruppen wie etwa Methoxy, Ethoxy und Propoxy ausgewählten Gruppen substituiert sein kann,
hydriert wird zum Erhalt der Verbindung der Formel II 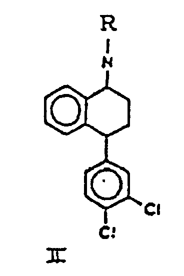
ii) die Verbindung der Formel II wird mit Formaldehyd oder einem Formaldehyd-Derivat reduktiv alkyliert zum Erhalt der Verbindung der Formel III, oder die Verbindung der Formel II wird mit einem Methylierungsmittel alkyliert zum Erhalt der Verbindung der Formel III 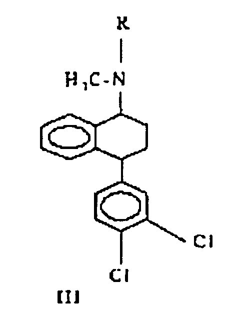
iii) die Verbindung der Formel III wird durch Entfernen der Gruppe R in Sertralin oder eines Salzes davon umgesetzt.

2. Verfahren nach Anspruch 1, wonach das Formaldehyd oder ein Formaldehyd-Derivat aus der Gruppe bestehend aus Formaldehyd, Paraformaldehyd, Acetalen und deren Halbacetalen ausgewählt wird.

3. Verfahren nach Anspruch 1, wonach das Methylierungsmittel Methyljod ist.

4. Verfahren nach einem der Ansprüche 1-3, wonach Stufe i) in Gegenwart eines aus Palladium auf Kohle, Rhodium auf Kohle und Raney-Nickel ausgewählten Katalysators durchgeführt wird.

5. Verfahren nach einem der Ansprüche 1-4, wonach Stufe i) und ii) in demselben Behälter ohne dazwischenliegende Läuterung durchgeführt werden.

6. Verfahren nach einem der Ansprüche 1-5, wonach Stufe iii) durch Hydrogenolyse durchgeführt wird.

7. Verfahren nach einem der Ansprüche 1-6, wonach R Benzyl ist.

8. N-substituiertes N-[4-(3,4-dichlorphenyl)-3,4-dihydro-1(2H)-naphthalenylidin)-amin der Formel I, worin R in Anspruch 1 definiert ist.

9. N-substituiertes N-[3,4-dichlor-phenyl)-1,2,3,4-tetrahydro-1-naphthalenamin der Formel II, worin R in Anspruch 1 definiert ist.

10. N-substituiertes N-methyl-4-(3,4-dichlorphenyl)-1,2,3,4-tetrahydro-1-naphthalenamin der Formel III, worin R in Anspruch 1 definiert ist.

## Revendications

1. Procédé pour la production de cis-(1S), (4S)-N-méthyl-4-(3,4-dichlorophényl)-1,2,3,4-tétrahydro-1-naphtylamine, sertraline, ou un sel de cela, **caractérisé en ce que** le procédé comprend les étapes suivantes :
i) le composé avec la formule I 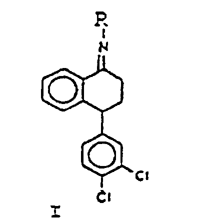 où R = un groupe benzylique qui peut être substitué par un à trois groupes choisis indépendamment parmi : fluorine, chlore, brome, iode, groupes d'alkyle tels que méthyle, éthyle et propyle, et groupes d'alkoxy tels que méthoxy, éthoxy et propoxy,
est hydrogéné pour obtenir le composé avec la formule II 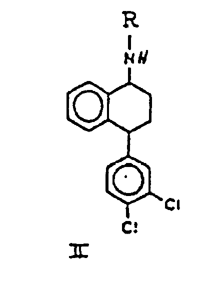
ii) le composé avec la formule II est alkylé de manière réductrice avec formaldéhyde ou un dérivé de formaldéhyde pour obtenir le compose avec la formule III, ou le composé avec la formule II est alkylé avec un agent de méthylation pour obtenir le composé avec la formule III 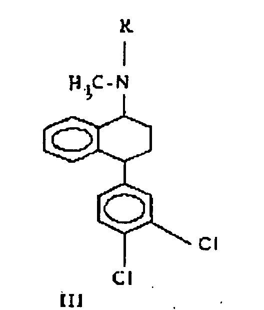
iii) le composé avec la formule III est converti à sertraline ou un sel de cela par la suppression de groupe R.

2. Procédé selon la revendication 1, où le formaldéhyde ou un dérivé de formaldéhyde est sélectionné du groupe constitué de formaldéhyde, paraformaldéhyde, acétals, et hémiacétals de cela.

3. Procédé selon la revendication 1, où l'agent de méthylation est iodure de méthyle.

4. Procédé selon l'une quelconque des revendications 1-3, où l'étape i) est effectuée en présence d'un catalyseur sélectionné de palladium sur carbone, rhodium sur carbone et nickel de Raney.

5. Procédé selon l'une quelconque des revendications 1-4, où l'étape i) et l'étape ii) sont effectuées dans le même vase sans purification intermédiaire.

6. Procédé selon l'une quelconque des revendications 1-5, où l'étape iii) est effectuée par hydrogénolyse.

7. Procédé selon l'une quelconque des revendications 1-6, où R = benzyle.

8. N-substitué N-[4-(3,4-dichlorophényl)-3,4-dihydro-1(2H)-naphtalènylidine]-amine de la formule I, où R est défini dans la revendication 1.

9. N-substitué N-(3,4-dichlorophényl)-1,2,3,4-tetrahydro-1-naphtylamine de la formule II, où R est défini dans la revendication 1.

10. N-substitué N-méthyl-4-(3,4-dichlorophényl)-1,2,3,4-tetrahydro-1-naphtylamine de la formule III, où R est défini dans la revendication 1.
